# EUROPEAN PATENT SPECIFICATION

(11) **EP 3 009 460 B1**
(45) Date of publication and mention of the grant of the patent: **05.12.2018**
(21) Application number: 14188808.1
(22) Date of filing: 14.10.2014
(51) Int. Cl.: C08G 18/48, C08G 18/61, C08G 18/64, C08G 18/08, C08G 18/10, C08G 18/12, C08G 18/42

(54) **Aqueous peptide-stabilized polyurethane dispersions**
Wässrige, peptidstabilisierte Polyurethandispersionen
Dispersions aqueuses de polyuréthane à lien peptidique stabilisé

(43) Date of publication of application: 20.04.2016
(73) Proprietor: Henkel AG & Co. KGaA, 40589 Düsseldorf (DE); Max-Planck-Gesellschaft zur Förderung der Wissenschaften e.V., 80539 München (DE)
(72) Inventor: Taden, Andreas, 40597 Düsseldorf (DE); Landfester, Katharina, 55122 Mainz (DE); Breucker, Laura, 51063 Köln (DE)

(56) References cited:
- EP-A1- 0 773 246
- US-A1- 2012 153 230

## Description

The present invention relates to aqueous peptide-stabilized polyurethane dispersions that can be used in various fields, such as adhesives and cosmetics, as they are surfactant and solvent free and have low VOC emissions. As hybrid materials, they provide for a versatile system that can be finely tuned and combine the advantageous properties of polyurethane and peptide materials. Also encompassed are processes for their production, compositions containing them and their use in cosmetics and adhesives.

The synthesis of peptide-polymer conjugates has sparked immense interest among researchers for many years. Peptides offer unique properties unattainably with synthetic polymers such as selective and specific interactions with other biomolecules, a highly defined hierarchical structure, as well as responsiveness to various external stimuli. Synthetic polymers, on the other hand, possess a high resistance to degradation and often bring to the table very interesting thermomechanical properties in films such as elasticity, glass transition, crystallinity, strength, etc. In order to combine the respective advantages of both natural and synthetic polymers, much effort has been directed towards the purification or laboratory synthesis of peptides and proteins, the synthesis of synthetic polymers with the desired properties, and the development of different coupling strategies of both natural and synthetic polymers.

Commonly, shorter peptides are synthesized through the sequential addition of amino acids using methods like the Merrifield solid-phase synthesis procedure, whereas longer proteins are commonly obtained by recombinant means, typically by expression in recombinant cells followed by cell lysis, protein purification and separation. Both of these general synthesis routes are laborious and costly and therefore unsuited for many applications where stringent control of peptide structure is not required. A promising alternative consists in obtaining mixtures of peptides through enzymatic cleavage or hydrolysis of naturally occurring proteins from wool, corn, soya, and other natural sources. These peptides are promising reagents for the synthesis of new materials from peptide-polymer conjugates.

As far as synthetic polymers are concerned, many different types of polymers have been investigated for the conjugation to peptides. One, so far little investigated, class of polymer particles are waterborne polyurethane dispersions (PUDs). PUDs can be synthesized from a wide variety of polyols by reacting them with polyisocyanates to yield water-dispersible polymers with very interesting properties in the films formed thereof, such as partial crystallinity, elasticity, and high modulus. The first step in the synthesis most often consists of a polyaddition reaction between the polyols and the polyisocyanate monomers to form a PU prepolymer. Commonly, polyols with charged groups and with water-swellable moieties are used in order to generate so-called "self-stabilizing" structures, i.e. which can be dispersed in water without the addition of a surfactant. Through addition of a slight excess of isocyanate with regard to alcohol functionalities, a prepolymer of moderate molecular weight with NCO end groups is obtained. These end groups can be used for a chain extension after dispersion to increase the molecular weight of the PU chains and/or for an end cap in order to introduce a desired functionality into the polymer structure.

Common coupling strategies to attach peptides to nanoparticles include simple adsorption, surface "click"-reactions, copolymerization, etc. European patent application EP 0 773 246 A1 describes the generation of graft polymers by reacting NCO-terminated polyurethanes with proteins, in particular casein. The graft polymers are synthesized by addition of an amino group containing protein to the polyurethane synthesis reaction mixture, as known in the art for termination of the polyurethane synthesis by addition of amines.

However, there exists still need in the art for an alternative general synthesis procedure for peptide modified polyurethane polymers, in particular PUDs.

The present invention is based on the inventors' finding of a general synthesis procedure to obtain PUDs with a peptide corona using a particularly facile route. More specifically, the inventors could demonstrate that - using a peptide mixture - careful control of the reaction conditions permits to obtain PUDs which do not only possess but are stabilized by a peptide corona. The invention therefore generally relates to a method to produce a dispersion of polyurethane particles with a peptide corona in water by applying shear forces. Especially when the peptide corona is the crucial component for the colloidal stabilization, it is expected that the biological functionality of the peptide can effectively govern the overall PUD properties. This way, weak external stimuli, like small changes in pH, temperature, ionic strength or the interaction with other molecules and specific surfaces, do not only influence the peptide in its pristine state but influence the whole peptide-PU conjugate. In fact, when the peptide attachment and is conformational state becomes critical for the PUD stabilization, the same weak external forces can drastically impact the stimuli-response of the overall peptide conjugate system. This critical peptide stabilization therefore is expected to be highly beneficial in a variety of applications, for example the deposition and adhesion to a variety of interfaces.

In a first aspect, the present invention thus relates to a process for manufacturing a peptide-stabilized polyurethane dispersion (PUD) comprising
(1) providing an NCO-terminated polyurethane prepolymer; and
(2) dispersing said NCO-terminated polyurethane prepolymer into a continuous aqueous phase, wherein the continuous aqueous phase is an aqueous solution comprising one or more peptides, thereby forming peptide-stabilized polyurethane particles.

In another aspect, the invention relates to the aqueous peptide-stabilized polyurethane polymer dispersion obtainable according to the process described herein.

Further aspects of the invention relate to compositions, in particular cosmetic and adhesive compositions, which contain the aqueous peptide-stabilized polyurethane polymer dispersion disclosed herein and the use of the aqueous peptide-stabilized polyurethane polymer dispersion in cosmetic and adhesive compositions.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more. "At least one", as used herein in relation to any component, refers to the number of chemically different molecules, i.e. to the number of different types of the referenced species, but not to the total number of molecules. For example, "at least one polyol" means that at least one type of molecule falling within the definition for a polyol is used but that also two or more different molecule types falling within this definition can be present, but does not mean that only one molecule of said polyol is present.

The term "peptide", as used herein, refers to a polymer of two or more amino acids that are bound to each other by peptide bonds. The peptides preferably comprise 2 or more amino acids, and are typically of a length of up to 1000, preferably up to 600 amino acids. In a preferred embodiment a peptide has an average molecular weight of between 1000 and 100.000 g/mol, more preferably between 1500 and 75.000 g/mol, and most preferred between 2000 and 60.000 g/mol. If reference is made herein to a molecular weight of peptides of > 10000 g/mol, this reference refers to the average number molecular weight determined via SDS-PAGE (sodium dodecyl sulfate polyacrylamide gel electrophoresis). If references are made to molecular weight of peptides of ≤ 10000 g/mol, the analytical method of choice in this size regime is size exclusion HPLC.

The amino acids of which the peptides are composed are preferably naturally occurring proteinogenic amino acids, such as the 20 amino acids making up naturally occurring proteins. Said amino acids include glycine, alanine, valine, leucine, isoleucine, phenylalanine, cysteine, methionine, proline, serine, threonine, glutamic acid, aspartic acid, asparagine, glutamine, lysine, arginine, histidine, tyrosine, and tryptophan. In various other embodiments, the amino acids making up the peptide can also comprise non-natural amino acids or non-proteinogenic amino acids.

If reference is made herein to a molecular weight of synthetic polymers, this reference refers to the average number molecular weight Mₙ, if not explicitly stated otherwise. The number average molecular weight Mₙ can be calculated based on end group analysis (OH numbers according to DIN 53240) or can be determined by gel permeation chromatography according to DIN 55672-1:2007-08 with THF as the eluent. If not stated otherwise, all given molecular weights are those determined by end group analysis. The weight average molecular weight M_{w} can be determined by GPC, as described for Mₙ.

All percentages given herein in relation to the compositions or formulations relate to weight % relative to the total weight of the respective composition or formula, if not explicitly stated otherwise.

In various embodiments of the processes, the aqueous solution comprises the one or more peptides in an amount of ≥4 wt.-%, preferably 4 to 20 wt.-%, more preferably 6 to 10 wt.-% relative to the total weight of the prepolymer. Lower amounts of peptide are typically insufficient to impart the desired stability to the polyurethane particles. Higher amounts become uneconomic, as the binding sites on the polyurethane particles become saturated. It has been found that amounts of about 7-10 wt.-%, preferably about 8 wt.-%, provide for stabilized dispersions over prolonged periods of time of more than 4 weeks in that they prevent agglomeration of the particles. In addition, these amounts provide for a narrow size distribution of the particles with a mean particle size of about 200 nm.

In various embodiments, the aqueous solution has a pH greater than the isoelectric point (IP) of the one or more peptides. In various embodiments, the one or more peptides are selected such that the isoelectric point of the one or more peptides is ≤7, preferably ≤6. If the peptides are a peptide mixture, the IP refers to the average IP of the mixture.

The one or more peptides may be a mixture of peptides. Such a mixture of peptides may be obtained by hydrolysis of a natural protein. The hydrolysis may be chemical hydrolysis or enzymatic hydrolysis, for example by use of protein-hydrolyzing enzymes, such as proteases. The natural protein that serves as a source for the peptides may be any suitable natural protein. Exemplary proteins that may be used include plant proteins, for example from corn and soya, or animal proteins, for example from wool, skin, hair, nails, horns, hooves, milk and the like. Preferred protein sources are insoluble fibrous proteins, for example keratins and collagen, which occur in hairs, nails, skin, connective tissue and the like. Purification and processing of such proteins, in particular keratins, followed by hydrolysis, yields peptide mixtures that are particularly suitable for the described purposes. It is understood that such a peptide mixture contains a variety of different peptides having different compositions and molecular weights. It is further understood that length and thus molecular weight of the peptides and thus also the average length and average molecular weight can be controlled by the hydrolysis technique and conditions. Preferred peptides have an average length of between 5 and 1000 amino acids, more preferably between 12 and 600 amino acids. In a particular preferred embodiment, hydrolyzed wool proteins are used, wherein said hydrolyzed wool proteins comprise a mixture of peptides of varying length having an average molecular weight of about 50.000 Da.

The peptides naturally have an amino-terminus, i.e. a free -NH₂ group, and a carboxy-terminus, i.e. a carboxylic acid or carboxylate group. In addition, depending on the composition of the peptide, the amino acid side chains comprise hydroxyl groups (serine, threonine, tyrosine), thiol groups (cysteine), carboxylic acid groups (aspartic acid, glutamic acid), amino groups (lysine), guanidine groups (arginine), carbamoyl groups (asparagine, glutamine), or imidazol groups (histidine). In various embodiments, at least one of these groups, typically an amino or hydroxyl group, reacts with the NCO-groups of the PU prepolymer that are accessible for the peptide and forms a covalent bond between the peptide and the PU prepolymer. The bonding between peptide and PU prepolymer can occur via one covalent bond only or via two or more covalent bonds, depending on the functionality of the peptide and spatial constraints. Alternatively or additionally, the peptides can adsorb to the surface of the PU prepolymer particles or droplets. Such adsorption is typically non-covalent and mediated by electrostatic or hydrophobic interactions, again depending on the composition of the peptide.

In various embodiments, the reaction between the PU prepolymer and the peptides during or upon dispersion of the prepolymer in the continuous aqueous phase typically generates PU particles with a peptide corona. "Peptide corona", as used herein, means that the PU particle is at its surface modified with peptides that are non-covalently or covalently bound and thus encase the particle surface in form of a corona. The peptides may adopt a structure such that their hydrophilic parts are exposed to the aqueous solvent while more hydrophobic parts are buried within the peptide fold or facing the PU particle surface.

In various embodiments of the invention, step (1) comprises forming an NCO-terminated polyurethane prepolymer from a reaction mixture comprising:
(a) at least one polyol, preferably with a number average molecular weight Mₙ in the range of 400 g/mol to 10000 g/mol; and
(b) at least one polyisocyanate, preferably at least one aliphatic di- and/or triisocyanate, wherein the at least one polyisocyanate is used in molar excess relative to the hydroxy groups of the at least one polyol of the reaction mixture to obtain an NCO-terminated polyurethane prepolymer.

The at least one polyol may be at least one polyester polyol, at least one polyether polyol, or at least one polycarbonate polyol or, preferably, a mixture of thereof, in particular a mixture of at least one polyether polyol and at least one polyester polyol. Also contemplated are thus mixtures of two or more polyester polyols and/or two or more polyether polyols. Further contemplated are mixtures of a polyester polyol and a polydimethylsiloxane (PDMS) polyol or of a polyester polyol, a polyether polyol and a PDMS polyol. If a mixture of polyester and polyether polyols or a mixture of polyester and PDMS polyols is used, the weight ratio may range from about 10:1 to 1:10, preferably 1:2 to 2:1. In various embodiments, the at least one polyol comprises at least one non-functionalized polyol, i.e. a polyol that contains no functional groups, such as anionic or cationic groups (e.g. carboxyl, sulfonyl, ammonium and the like) besides the hydroxyl groups.

Polyester polyols that are useful in the processes described herein include those that are obtainable by reacting, in a polycondensation reaction, dicarboxylic acids with polyols. The dicarboxylic acids may be aliphatic, cycloaliphatic or aromatic and/or their derivatives such as anhydrides, esters or acid chlorides. Specific examples of these are succinic acid, glutaric acid, adipic acid, pimelic acid, suberic acid, azelaic acid or sebacic acid, phthalic acid, isophthalic acid, trimellitic acid, phthalic acid anhydride, tetrahydrophthalic acid anhydride, glutaric acid anhydride, maleic acid, maleic acid anhydride, fumaric acid, dimeric fatty acid and dimethyl terephthalate. Examples of suitable polyols are monoethylene glycol, 1,2-propanediol, 1,3-propanediol, 1,4-butanediol, 3-methylpentane-1,5-diol, neopentyl glycol (2,2-dimethyl-1,3-propanediol), 1,6-hexanediol, 1,8-otaneglycol cyclohexanedimethanol, 2-methylpropane-1,3-diol, dithyleneglycol, triethyleneglycol, tetraethyleneglycol, polyethyleneglycol, dipropyleneglycol, polypropyleneglycol, polypropyleneglycol, dibutyleneglycol and polybutyleneglycol. Alternatively, they may be obtained by ring-opening polymerization of cyclic esters, preferably ε-caprolactone.

In various embodiments, the polyester polyol has a melting temperature Tₘ > 0°C, preferably > 30 °C, more preferably > 40 °C and/or has an average number molecular weight Mₙ in the range of 400 to 5000, preferably 500 to 3000 g/mol, more preferably 800-2500 g/mol, most preferably 1000 to 2000 g/mol.

The polyether polyol may be a polyalkylene glycol homo- or copolymer, preferably a polypropylene glycol homo- or copolymer, a polyethylene glycol homo- or copolymer, a polytetramethylene glycol homo- or copolymer, a polypropylenglycol/polyethyleneglycol block copolymer.

In various embodiments, the polyether polyol has an average number molecular weight of 1000 to 4000, preferably 1000 to 3000 g/mol.

Suitable polycarbonates can be obtained by reaction of carbon acid derivatives, e.g. diphenyl carbonate, dimethyl carbonate or phosgene with diols. Suitable examples of such diols include ethylene glycol, 1,2- and 1,3-propanediol, 1,3- and 1,4-butanediol, 1,6-hexanediol, 1,8-octanediol, neopentyl glycol, 1,4-bishydroxymethyl cyclohexane, 2-methyl-1,3-pro-panediol, 2,2,4-trimethyl pentanediol-1,3, dipropylene glycol, polypropylene glycols, dibutylene glycol, polybutylene glycols, bisphenol A, tetrabromobisphenol A as well as lactone-modified diols. The diol component preferably contains 40 to 100 wt. % hexanediol, preferably 1,6-hexanediol and/or hexanediol derivatives. More preferably the diol component includes examples that in addition to terminal OH groups display ether or ester groups.

The hydroxyl polycarbonates should be substantially linear. However, they can optionally be slightly branched by the incorporation of polyfunctional components, in particular low-molecular polyols. Suitable examples include glycerol, trimethylol propane, hexanetriol-1,2,6, butanetriol-1,2,4, trimethylol propane, pentaerythritol, quinitol, mannitol, and sorbitol, methyl glycoside, 1,3,4,6-dianhydrohexites.

Suitable polycarbonate polyols are, without limitation, those obtainable under the trademark names Desmophen® C3200 (Bayer) and Kuraray® C2050 (Poly-(3-methyl-1,5-pentanediol, 1,6-hexanediol)carbonate; Kuraray).

In various embodiments, the reaction mixture includes:
(a) at least one crystalline or semicrystalline polyol, preferably polyester polyol, with a number average molecular weight Mₙ in the range of 400 g/mol to 5000 g/mol, a crystallinity of at least 20 % , preferably 35 % and more preferably 50% and a melting temperature Tₘ in the range of 30°C to 150°C, preferably 40 - 120 °C and more preferably 40 - 80 °C the crystallinity and the melting temperature as determined by differential scanning calorimetry (DSC) according to ISO11357; and optionally
(b) at least one amorphous polyol, preferably polyether polyol, with a number average molecular weight Mₙ in the range of 400 g/mol to 5000 g/mol, a crystallinity of less than 10 % and a glass transition temperature T_{g} in the range of -60°C to 40°C, preferably -40°C to 30°C, the crystallinity and the glass transition temperature as determined by differential scanning calorimetry (DSC) according to ISO11357, wherein the molar ratio of the polyol (a) to the polyol (b) is in the range of 1:10 to 10:1.

The amorphous polyols that can be used in accordance with the embodiments described herein are preferably polyether polyols, in particular polypropylenglycol (PPG) or copolymers of propylenglycol and polyethylenglycol and/or have a number average molecular weight Mₙ in the range of 400 g/mol to 5000 g/mol, preferably 500 to 3000 g/mol, more preferably 800-2500 g/mol, most preferably 1000 to 2000 g/mol.

"Amorphous", as used herein in relation to the (polyester) polyols, means that the (polyester) polyol has a crystallinity of less than 10 %, preferably less than 5 %, more preferably less than 2 %, as determined by differential scanning calorimetry (DSC) according to ISO11357. The amorphous (polyester) polyols furthermore have a glass transition temperature T_{g} in the range of -60°C to 40°C, preferably -40°C to 30°C, again as determined by differential scanning calorimetry (DSC) according to ISO11357. Below the glass transition temperature, amorphous polymers are brittle and rigid. This property is due to the immobility of the "frozen" polymer chains. When the glass transition temperature is exceeded the molecular chains become movable relative to one another and the polymer softens, the degree of softening depending on the type of the polymer, the molecular weight of the polymer and the temperature. Amorphous polymers, as compared with (semi-)crystalline polymers, show only a glass stage in the DSC measurement according to ISO11357 during the transition from the more brittle, rigid state to the softened state. A melt peak indicating a semi-crystallinity of the polymer does not occur in the DSC measurements.

The crystalline or semicrystalline polyol is preferably a polyester polyol and may have a number average molecular weight Mₙ in the range of 400 g/mol to 5000 g/mol, preferably 500 to 3000 g/mol, more preferably 800-2500 g/mol, most preferably 1000 to 2000 g/mol. "Crystalline", as used herein in relation to the (polyester) polyols, relates to a crystallinity of at least 90%, preferably at least 95%, as determined by differential scanning calorimetry (DSC) according to ISO11357. Similarly, "semicrystalline", as used herein in relation to the (polyester) polyols, means that they have a crystallinity of at least 20 %, preferably at least 35 % and more preferably at least 50%, but less than 90%. Semicrystalline (polyester) polyols thus comprise crystalline and non-crystalline, i.e. amorphous, regions.

The crystalline and semicrystalline (polyester) polyols useful in the described mixtures may have a melting temperature Tₘ in the range of 30°C to 150°C, preferably 40 - 120 °C and more preferably 40 - 80 °C, again as determined by differential scanning calorimetry (DSC) according to ISO11357.

In embodiments, where a combination of amorphous (polyether) polyols and crystalline/semicrystalline (polyester) polyols is used, the molar ratio of the (polyether) polyol to the (polyester) polyol may be in the range of 1:10 to 10:1, preferably 1:3 to 5:1, more preferably 1:2 to 2:1.

The reaction mixture may comprise further polyols, in particular diols. Such diols may be monomeric diols, such as 1,4-butanediol.

The reaction mixture may further comprise polyols that are hydroxy-functionalized polymers, for example hydroxy-functionalized siloxanes. Exemplary siloxanes that may be used are hydroxy-functionalized polydimethylsiloxanes, in particular in liquid form, such as those commercially available under the name Tegomer® H-Si 2311 (Evonik, Germany) having a molecular weight Mn of about 2200 g/mol. In various embodiments, these are combined with the semicrystalline/crystalline polyester polyols described above and/or may be used in addition or instead of the amorphous polyols described above. Suitable polydimethylsiloxane (PDMS) polyols are, for example, described in US 6794445 B2. They may be used in amounts of up to 60 wt.-% based on the total weight of the polyols used and typically have low T_{g} values, for example in the range of from -150 to -100 °C.

In some embodiments, the reaction mixture may further comprise at least one stabilizer, said stabilizer being different from the peptides used. It is however preferred that the reaction mixture does not contain such a stabilizer, as the stabilizing effect is provided by the peptides upon dispersion into the aqueous phase.

The term "stabilizer", as used herein, relates to a class of molecules that can stabilize the hydrophobic droplets in a water-based (oil-in-water, abbreviated as o/w) dispersion or emulsion, i.e. prevent coagulation or coalescence. The stabilizer molecules may bind to, adhere to or associate with the droplet surface. In various embodiments of the invention, the peptides act as the stabilizer molecules, preferably as the sole stabilizer molecules. The term "stabilizer", as used herein, preferably relates to molecules that have a HLB value > 10, preferably > 12 and more preferably >15 according to Griffin and/or a water solubility of > 10 g/L, preferably > 50 g/L, more preferably > 100 g/L and most preferably > 150 g/L. Furthermore species which are able to carry an electric charge in water in a pH regime between pH3 (±1) to pH 11 (±1) are considered as stabilizers. Examples are molecules containing an anionic or latent anionic moiety, e.g. sulfate, sulfonate, phosphate, phosphonate or carboxylic groups without being limited to these. Additionally molecules containing cationic or latent cationic groups, like quaternary or tertiary amine groups, without being limited to these, are also encompassed by the term "stabilizer".

In a preferred embodiment of the invention, the peptides are essential for stabilization and/or act as the sole stabilizer molecules. This may mean that the PUD is essentially free of other stabilizer molecules and the method uses no stabilizers aside from the peptides.

In this context, "stabilization" or "essential for stabilization" relates to the property of the peptides to provide colloidal stabilization to polyurethane (pre)polymer systems in water, which would, in the absence of the peptide and even when high shear forces are applied (which can support the stabilization process by enabling smaller particle sizes and hence reducing buoyancy effects), not be colloidally stable in water.

In various embodiments, the use of additional stabilizer molecules, apart from the peptide stabilizing species, is therefore limited to amounts that do, in the absence of the peptides, not suffice to obtain polyurethane-based o/w-emulsions or dispersions with colloidal stability, even when high shear forces are applied.

The term "colloidal stability", as used herein, relates to emulsions or dispersions which do not change their average particle size by more 100 %, preferably not more than 50 %, more preferably not more than 25 % and most preferably not more than 10 % over 28 day of storage. The storage conditions are room temperature without agitation and without direct light exposure and at a solid contents of at least 5 %, preferably at least 10 %, more preferably at least 20 % and most preferably more than 30 % by weight. The pH and the ionic strength remain unchanged after emulsification and no other components, especially no surface active components for stabilization, no rheology agents or solvents are added. However, if necessary, as an exemption a biocide agent might be added upon storage in concentrations of up to 0,5 % (e.g. Acticide MBS, Thor company) For the particle size determination laser diffraction (LD) measurements as described in manuscript below can be applied. Alternatively Dynamic Light Scattering (DLS) can be used, as defined by the z-average according to ISO 22412:2008, when the starting particle size is below 500 nm,

Generally, stabilizer molecules as known in the prior art comprise a hydrophilic and a hydrophobic part, with the hydrophobic part interacting with the droplet and the hydrophilic part be exposed to the solvent. Commonly used stabilizers are surfactants and may bear an electric charge, for example may be anionic surfactants or cationic surfactants, or may, alternatively, be non-ionic.

However, to avoid the use of external surfactants and also due to the increased stability provided, recently reactive stabilizer compounds that are built into the polyurethane polymer during (pre)polymer formation have become more popular, as these provide for (self-)emulsifiable polyurethanes which under agitation, e.g. high sheer emulsification, or spontaneously form stable dispersions in water without the assistance of external emulsifiers.

These reactive stabilizers comprise or consist of isocyanate-reactive functionalities in order to become incorporated in the polyurethane structure. Preferred species are polyols, most preferably diols, or a mixture of different polyols and/or diols. Non-ionic polymeric stabilizers are known, with an average molecular weight Mₙ of about 4000 g/mol, preferably up to about 3000 g/mol, more preferably up to about 1000 g/mol. Suitable non-ionic ethylene glycol/propylene glycol stabilizers are for example those commercially available under the trademark name Pluronic® from BASF, for example Pluronic PE3500. The non-ionic stabilizers may, in various embodiments, have HLB (hydrophile lipophile balance) values between 6 and 20, preferably > 10. The HLB values are calculated by calculating the hydrophilic portion of the molecule by dividing the molecular weight of the hydrophilic part of the molecule, such as the alkylene oxide, by the total molecular weight of the molecule and then dividing the obtained percentage by 5.

While the afore-mentioned stabilizers are non-ionic, they may be modified by anionic or cationic groups. The presence of such charged groups may increase the stability of the dispersed polymer droplets or particles. Suitable anionic groups include, but are not limited to acidic groups, such as carboxylic acid or sulfonic acid groups and their respective salts. Concrete compounds suitable as anionic stabilizers in the sense of the present invention are 2,2-bis(hydroxymethyl)propionic acid (dimethylol propionic acid; DMPA) and sulfonated polydiols with a molecular weight M_{w} in the range of up to 1000 g/mol, preferably up to 500 g/mol. Such sulfonated polydiols, for example propoxylated 1-methyl-2-methylol-3-hydroxy-1-propanesulfonate with a molecular weight M_{w} of about 430 g/mol, are commercially available under the name GS-7Q (Yedang G & Co. Ltd).

Suitable cationic groups include, but are not limited to ammonium groups, in particular quaternary ammonium groups. Particularly preferred are alkoxylated, preferably ethoxylated or propoxylated, dialkylammonium diols, such as ethoxylated cocoalkylmethyl ammonium methanesulfonate. Such compounds are commercially available under the trademark name Rewoquat® CPEM from Evonik.

It is however preferred that such ionic stabilizers that provide for self-stabilization of the PU particles are not used in the reaction mixtures of the invention, at least not amounts that allow effective colloidal stabilization in the absence of the peptides.

The term "reaction mixture", as used herein, relates to the mixture of the polyols and the polyisocyanate(s). "Polyol mixture", as used herein in relation to the mixture comprising the polyols, relates to a mixture comprising the at least one polyol and, optionally, any additional polyols, including polyhydroxy-modified polymers of various types, that may be present.

It is preferred that the polyol mixture does not contain any organic solvents or surfactants and no further additives, i.e. consists only of the polyols, preferably those defined above.

In various embodiments, the polyol mixture comprises about 20 to about 100 wt.-%, preferably 30 to 100 wt.-%, of the at least one polyol, preferably a mixture of different polyols, for example of polyester polyols and polyether polyols, relative to the weight of the polyol mixture. If present, the stabilizer is usually contained in amounts of about 1 to 30 wt.-%, preferably 10 to 20 wt.-%, relative to the weight of the polyol mixture.

The other reactant employed in the formation of the polyurethane prepolymer is a polyisocyanate. Any compound which includes at least two isocyanate groups is within the contemplation of the present invention. It is preferable, however, that the polyisocyanate be a diisocyanate. The incorporation of small amounts of isocyanate with a functionality higher than two, in particular a triisocyanate, is also contemplated and may under certain circumstances even be advantageous. Such polyisocyanates can act as cross-linkers. In this case where the polyisocyanate acts as a crosslinker, polyisocyanates based on hexamethylene diisocyanate are preferred. Suitable diisocyanates include, without limitation, methylenediphenyl diisocyanate (MDI), toluene-2,4-diisocyanate (TDI), hexamethylene diisocyanate (HDI), polymeric diphenylmethane diisocyanate (PMDI), isophorone diisocyanate (IPDI), methylene-4,4-bis(cyclohexyl)diisocyanate (H12MDI) and mixtures thereof. Although both aliphatic and aromatic polyisocyanates are within the contemplation of the present invention, it is preferred that the polyisocyanate be an aliphatic polyisocyanate. Thus, in a particularly preferred embodiment, the polyisocyanate is an aliphatic diisocyanate. Among particularly preferred aliphatic diisocyanates are isophorone diisocyanate, hexamethylene diisocyanate, and mixtures thereof. Suitable polyisocyanates are, for example, commercially available under the trademark name Desmodur® from Bayer AG (DE).

The polyisocyanate is used in molar excess relative to the OH groups of all polyols present in the reaction mixture, the OH/NCO equivalent ratio preferably being 1:1.1 to 1:4, more preferably 1:1.2 to 1:1.3. The amount of the at least one polyisocyanate in the reaction mixture is typically in the range of 10 to 20 wt.-% relative to the reaction mixture. The remainder of the reaction mixture may be made up by the polyol mixture, as defined above.

Providing the polyol mixture may include the step of mixing the polyols and heating the mixture. The heating may be required in case the polyols employed are solid at room temperature and need to be melted to form the polyol mixture. In preferred embodiments, the polyols are combined and heated to about 70 to 95°C, for example about 75°C, while stirring the mixture under vacuum to dry. After the mixing, the mixture may be cooled to 60°C for the addition of the isocyanates.

The polyol mixture is subsequently combined with at least one polyisocyanate in the reaction mixture to form the prepolymer. The prepolymer reaction usually occurs at elevated temperature, preferably in the range of between about 70 °C and about 95 °C, more preferably about 80 °C, over a period of between about 1 and about 24 hours. The reaction is typically carried out in the presence of a catalyst that is added, preferably a tin-, bismuth- or zinc-based catalyst, more preferably dimethyldineodecanoatetin, for example commercially available under the trade name Fomrez UL28 (Momentive Performance Materials GmbH, Germany). Alternative catalysts of high reactivity are Bismuth-neodecanoate and Zn-Neodecanoat available under the trade names BorchiKat 315 and BorchiKat 0761 (OMG Borchers GmbH, Germany). In preferred embodiments of the invention, the reaction mixture thus further comprises a catalyst as defined above.

The reaction continues until the free isocyanate content reaches or comes very close to the calculated value, as determined by standard titration with dibutylamine. Preferred values for the free isocyanate content in the prepolymer are in the range between 0.2 and 3 wt.-%, preferably 1 to 2 wt.-% relative to the total amount of polyols, including the stabilizer(s), and polyisocyanate in the mixture.

"About", as used herein, relates to ± 10 %, preferably ± 5 % of the numerical value to which it refers. "About 70 °C" thus relates to 70 ± 7, preferably 70 ± 3.5 °C.

As described above, in the polyurethane prepolymer-forming reaction the polyisocyanate is reacted in a concentration in excess of the stoichiometric concentration required to completely react with the hydroxyl groups. The excess used may include an OH/NCO equivalent ratio of 1:1.1 to 1:4. Preferably, the amount of the polyisocyanate is 20 % to 150 % in excess of the stoichiometric concentration required to completely react with the hydroxyl groups.

Once the free isocyanate content reaches the predetermined value, as defined above, the temperature may be reduced, for example to about 60 °C. Subsequently, the formed prepolymer may be dissolved in a suitable organic solvent, including, without limitation, ethyl acetate or acetone.

In various embodiments, the prepolymer has an average number molecular weight Mₙ of 3000 to 12000, preferably 4000 to 6000 g/mol.

In various embodiments, the prepolymer comprises crystalline segments, e.g. has a crystallinity of at least 20 % or more, preferably at least 35 %, more preferably at least 50 %. The crystallinity can be determined as described above, e.g. by differential scanning calorimetry (DSC) according to ISO11357 at a heating rate of 10 K/min. In case the crystallinity is tested, it should be done with the prepolymers of low molecular weight before emulsification and/or chain extension. It is preferred that the prepolymers are endcapped for this purpose with a suitable end-capping agent, e,g, dibutylamine. The results for crystallinity should be compared with the literature values for the polyols applied, or, more preferred, validated by x-ray measurements. Because the degree of semi-crystallinity for a given polymer is depending on the thermal history, the polymeric and semi-crystalline materials should be staged for at least 24 hours, preferably a week at a temperature of five Kelvin below the respective melting temperature as defined via DSC according to ISO11357.

In various embodiments and if necessary, the prepolymer may be neutralized at this stage by using a suitable neutralization agent. Typically, this is only necessary if an ionic stabilizer is used. For example, in case an anionic acidic stabilizer is used, an amine base, such as triethylamine may be used for neutralization.

The thus formed prepolymer is then dispersed in the continuous aqueous phase containing the peptides. The aqueous phase preferably contains as a solvent predominantly (>50 vol.-%, preferably >80 vol.%, more preferably >90 vol.-%) or exclusively (i.e. 100 vol.-%) water, i.e. contains no other liquid solvent. The aqueous phase may however further comprise solutes, in particular the peptides but also, if desired, salts and buffer substances.

The dispersion may be achieved under vigorous agitation. In various embodiments, the prepolymer is used in form of a solution of the prepolymer in an organic solvent, with the solution then being emulsified in water. The dispersing step may be carried out at room temperature or at elevated temperature, for example in the range of about 20 to 60 °C, for example at about 40 °C. The dispersing step may be done by mechanical stirring alone or by dispersing devices, such as high-pressure homogenizers, microfluidizers or rotor-stator dispersing machines, such as an ultra-turrax device.

The dispersing step may include emulsifying the polyurethane prepolymer in liquid form into a continuous aqueous phase, preferably water, to form a pre-emulsion; and homogenizing the pre-emulsion to form a stable miniemulsion. Homogenization of the pre-emulsion in order to form a miniemulsion may be achieved by the action of a shear force, preferably under application of high shear forces, for example by a high-pressure homogenizer. The homogenizer may have an energy input in the range of from 10³ to 10⁵ J per second per liter of the emulsion.

Generally, the dispersing step, preferably the homogenization step, may be carried out with a high shear process. Preferably, a high shear process relates to application of high shear forces, wherein high shear forces is to be understood as to apply (a) shear rates of at least 1.000.000/s, and/or (b) an energy input per time of at least 10³ J per second per liter of the emulsion.

For the dispersing step, the prepolymer is preferably used in liquid form, i.e. it is either liquid at room temperature or, if it is solid at room temperature, it is melted or dissolved in a suitable organic solvent, such as, without limitation, acetone or ethyl acetate. If an organic solvent is used, this may be removed later from the dispersion by known means, for example by a rotary evaporator.

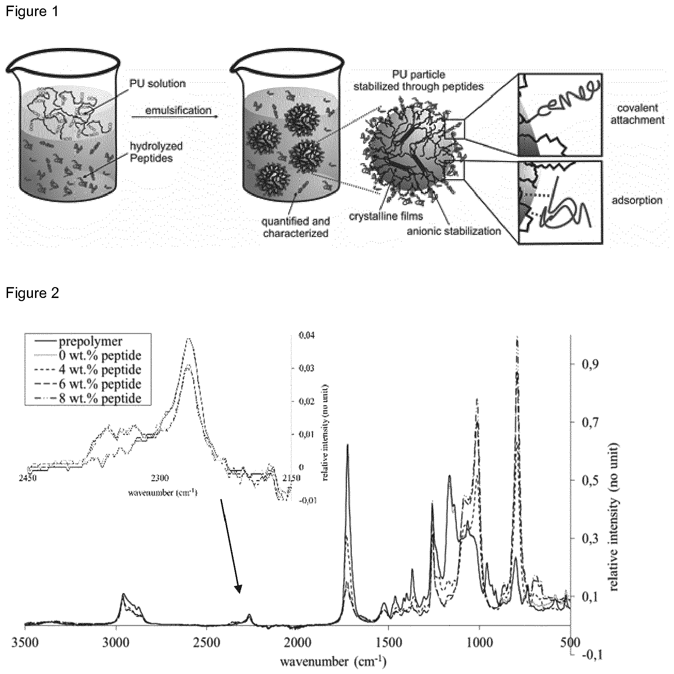

The term "miniemulsion" or "emulsion", as used herein, relates to oil-in-water (O/W) emulsions, i.e. emulsions in which water is used in excess and is the continuous medium. In the described processes, stable droplets/particles of the PU prepolymer with a peptide corona are obtained, which have typically a size between 50 and 500 nm, preferably between 100 and 400 nm. The emulsion process is schematically shown in **Figure 1**.

In addition to the reaction with the peptides, remaining isocyanate end-groups of the prepolymer may be reacted with an appropriate chain extender containing at least two terminal NCO-reactive groups, for example a diamine, such as hydrazine, an alkylene diamine or cycloalkylene diamine, preferably ethylene diamine, isophorone diamine, piperazine, or polyetheramine. Diols, such as an alkyldiol, including but not limited to 1,4-butanediol and 2-butyl-2-ethyl-1,3-propanediol, or water can also be used. The chain extension reaction may be performed until essentially total conversion of the isocyanate groups, i.e. the chain extension agent is continuously added until free isocyanate groups are no longer detectable. It is generally preferred that the chain extension reaction is carried out until total conversion of the isocyanate groups. The conversion can be monitored by techniques well-established in the art, for example IR spectroscopy. However, in a preferred embodiment the stabilizing peptides, which can covalently react with the polyurethane prepolymers on multiple sites, are used as the sole chain extension agent. This ensures that the PU prepolymer particle surface is saturated with peptide moieties. The presence of a catalyst and higher temperature may also be required.

The processes of the invention may further comprise a step of (3) separating the peptide-stabilized polyurethane polymer particles from the unreacted peptides. This may for example be achieved by dialysis or any other suitable technique, such as chromatography.

As already described above, the peptide-stabilized polyurethane polymer particles are polyurethane particles having a peptide corona. As the PU prepolymer may be synthesized without any charged monomers or building blocks and the dispersion may also contain no additional stabilizers, the stabilization may be exclusively provided by the peptide moieties coupled/adsorbed to the particle surface, i.e. the peptide corona. Accordingly, in various embodiments, the polyurethane prepolymer is essentially free of electrically charged groups or electrically charged monomeric units. Stabilization by means of the peptide corona and the possible omission of the use of stabilizing units in the prepolymer also allows the use of prepolymers having a certain degree of crystallinity, as described above.

"Essentially free" or "essentially no", as used in this context, means that the dispersion and/or composition contains less than 5 wt.-% of the given component, preferably less than 2 wt.-%, more preferably less than 1 wt.-%.

The aqueous polyurethane dispersion formed preferably has a solid content of 5 to 70 wt.-%, preferably 15 to 60 wt.-%, more preferably 20 to 55 % and most preferably 30 -50 %.

As already mentioned above, the peptide-stabilized polyurethane particle dispersions obtainable by the described processes form also part of the present invention.

The aqueous peptide-stabilized polyurethane dispersions may be used in various fields, such as, without limitation, cosmetic and therapeutic products and compositions, adhesive applications and corrosion protection. Adhesive applications include laminating applications for substrates such as leather (shoes) and wood. Such compositions can contain further ingredients all of which are well-known in the field. Accordingly, such uses as well as the compositions as such, in particular cosmetic compositions, such as, without limitation, hair care products, are also encompassed by the present invention.

It is understood that all embodiments disclosed herein in relation to the processes and methods are similarly applicable to the disclosed dispersions, compositions, and uses, and vice versa.

All documents cited are herein incorporated by reference in their entirety.

The following examples are given to illustrate the present invention. Because these examples are given for illustrative purposes only, the invention should not be deemed limited thereto.

### Examples

### Materials and Methods

### Chemicals

The poly(oxypropylene/oxyethylene) glycol (Tergitol L-61, M = 2117 g/mol) was obtained from Dow Chemical Canada, Inc., Canada. The polyester resin (Realkyd XTR 20112, M = 2117 g/mol) was obtained from Arkema Coatings Resins Sri, Italy. Alcohol-terminated Polydimethyl Siloxane (Tegomer H-Si 2311, M = 2200 g/mol) was obtained from Evonik Industries AG Essen, Germany. Isophorone diisocyanate (IPDI, M = 222.29 g/mol) was obtained from Sigma-Aldrich Chemie GmbH, Germany. The tin-based catalyst (Fomrez UL-28) was obtained from Momentive Performance Materials GmbH, Germany. The hydrolyzed peptide mixture Keratec IFP PE was supplied by Croda GmbH Germany and freeze-dried before use. The defoamer Foamaster 223 was obtained from Cognis GmbH Germany. Ethyl acetate (EA) was obtained from Sigma-Aldrich Chemie GmbH Germany and dried with molecular sieve. All other chemicals were used as received.

### FTIR

FTIR measurements were taken using an Alpha FT-IR Spectrometer from Bruker Optik GmbH, Ettlingen Germany. It was operated in Platinum ATR single reflection diamond ATR module. Prior to the measurement, each sample taken was dried in a vacuum-oven for 30 min at room temperature to remove water.

### BCA Assay

To perform the BCA Assay, 10 ml samples from each batch of dialysis water (1.5 h, 3 h, 18 h, 42 h, 66 h, 168 h) from each synthesis (0 wt.%, 4 wt.%, 6 wt.%, and 8 wt.% peptide) freeze-dried and redissolved in 100 µl of deionized water. The same was done with control samples containing 1.25x10⁻³ wt. %, 2.50x10⁻³ wt.%, 7.50 x10⁻³ wt.%, 1.5 x10⁻² wt.%, 3.0 x10⁻² wt.%, 7.50 x10⁻² wt.%, and 1.0 x10⁻¹ wt.% peptide in deionized water. The BCA working solution was prepared by adding 25 ml of reagent 1 and 500 µl of reagent 2. Of each solution to be analyzed, three times 25 µl were taken and poured into a 96-well plate. 200 µl of working reagent were added and the plates were incubated at 40 °C for 1h. This was also done with three times 25 µl of deionized water for comparison. Fluorescence intensity was measured at 590 nm using a Tecan M1000 infinite plate reader from Tecan Group Ltd., Männedorf, Switzerland.

### Zeta Potential

Zeta Potential measurements were carried out using a Zetasizer Nano ZS from Malvern Instruments Ltd., Malvern, United Kingdom. In order to avoid interference from non-attached peptides, samples after dialysis were measured. Only the sample without peptides was measured right after synthesis because it did not remain stable thereafter.

### Laser Diffraction (LD)

LD measurements were carried out using a Horiba LA-950 Particle Analyzer from Retsch Technology GmbH, Haan, Germany. Samples from each synthesis (using 0 wt.%, 4 wt.%, 6 wt.%, and 8 wt.% peptide) were measured three times every week for one month after synthesis.

### Transmission Electron Microscopy (TEM)

For TEM sample preparation, a drop of the diluted dispersion containing 8 wt.% peptide was applied on a carbon-coated grid. Water was evaporated at RT under the hood. TEM images were obtained with a Tecnai F20 (FEI Deutschland GmbH, Frankfurt, Germany) operated at an acceleration voltage of 200 kV.

### Differential Scanning Calorimetry (DSC)

For DSC experiments, films were made from the samples containing 0 wt.%, 4 wt.%, 6 wt.%, and 8 wt.% of peptide and of the prepolymer before dispersion. The films were stored at 70 °C for 1 h, then at 5 °C for 1 h, and at 40 °C for 3 days in order to permit for melting of crystalline domains, crystal nucleation and growth, respectively.

### Example 1: Synthesis of PUDs

### Synthesis of PU prepolymer

Tergitol L-61 (12.50 g ; 5.905 mmol), Realkyd XTR 20112 (35.21 g ; 16.63 mmol), and Tegomer H-Si 2311 (62.50 g ; 28.41 mmol) were heated to 80 °C until molten, then vacuum (P < 0.1 mbar) was applied for 1 h to remove traces of water. N₂ was fed into the flask and the temperature was allowed to drop to 70 °C. IPDI (14.795 g; 66.557 mmol) was added. Under nitrogen atmosphere and reflux, the reaction was catalyzed by addition of Fomrez-UL-28 (0.01 g). After the initial increase in temperature, the reaction was carried out at 80 °C. The NCO content was checked every half hour using the Back Titration Method until it had reached the theoretical residual value of 1.05 % (2 h).

### Characterization of the Peptide Mixture Keratec IFP PE

In order to ensure the reactivity of the different amino acid residues present in peptide mixture towards the NCO end groups in the PU, the peptide mixture was first analysed using isoelectric focusing (IEF-PAGE). It was determined that the hydrolyzed peptide mixture consists of peptides with isoelectric points (IPs) of 3.9, 6.0, and of peptides with IPs between 4.2 and 4.8. In order to ensure that a large quantity of residues was available to react with the NCO end groups in the polymer, a pH well above the highest IP in the peptide mixture was chosen for the dispersion water, namely a pH of 6.5 to 6.8. These pH values were reached when the hydrolyzed peptide was dissolved in water such that no additional acid or base was added.

A careful control of the pH is primordial when coupling peptides to a PU polymer using the reactive NCO end groups because NCO groups are generally capable of reacting with any kind of nucleophile, including water. Thus, when the PU comes in contact with an aqueous solution of peptides, the reactive groups in the peptide are in competition with the water molecules for reaction with the isocyanate groups. In order to favour the reaction of the NCO groups with nucleophiles in the peptides over a reaction with the water molecules, the coupling was done at a pH between 6.5 and 6.8 where the peptides possess an overall negative charge, thus most of the residues present are deprotonated and reactive towards an isocyanate. Furthermore, coupling was done directly during the dispersion step (as opposed to later, when the dispersion was already formed), as described in the following, because the high shear forces employed further favour the reaction of the large and possibly sterically hindered peptides with the NCO groups.

### Dispersing

Dried ethyl acetate (125 g) was added to the prepolymer under vigorous stirring and reflux. From the prepolymer solution, four batches of 35.5 g each were taken. To each solution, one drop of defoamer was added. Three solutions of peptide were prepared containing 0.7098 g, 1.0647 g, and 1.4196 g of peptide (corresponding to 4, 6, and 8 wt.% relative to PU prepolymer, respectively) and deionized water was added to each for a total respective weight of 70.0 g. For the negative control, 70.0 g of deionized water were prepared. The amount of continuous phase was chosen such that after dispersion, the initial concentration of NCO in the system was 0.175 wt.%. The prepolymer solutions and the peptide solutions were heated to 40 °C. Then, four dispersions were formed using an UltraTurrax operating at 13 000 rpm for 10 min. Each dispersion was then passed through a homogenizer four times.

### Dialysis

In order to separate the non-attached peptides from the dispersion, dialysis was performed after dispersion. According to the manufacturer's information, the peptide contains fragments of rather high molecular weight (up to 50 000 g/mol). Thus, a dialysis membrane made from cellulose ester with a Molecular Weight Cut Off (MWCO) of 100 kDa was obtained from Carl Roth GmbH & Co. KG, Karlsruhe, Germany. Prior to dialysis, the membrane was cut into 4 pieces of 25 cm length approximately which were soaked in deionized water for 30 min in order to remove the preservative. Each piece of membrane was filled with 40.00 g of one of the dispersions (containing 0 wt.%, 4 wt.%, 6 wt.% or 8 wt.% Peptide) and closed off with clamps. Four dialysis baths containing 4000.00 g of deionized water were prepared and dialysis was carried out for one week in total. The dialysis water was changed after 1.5 h, 3 h, 18 h, 42 h, 66 h, and 168 h (1 week). Samples of the dialysis water were held back in order to permit quantifying the peptide flushed out.

### Example 2: Analysis of the PUD

### Investigation of the mode of attachment and quantification of the attached peptide

After IEF investigations and identification of the working pH range of 6.5 to 6.8, a PU prepolymer was synthesized, dissolved in ethyl acetate and dispersed in different concentrations of peptide (0 wt.%, 4 wt.%, 6 wt.%, and 8 wt.%) with regard to the prepolymer as described in Example 1.

In order to assess how much of the peptide mixture was covalently bound to the PUD, how much was adsorbed to the particles, and how much did not interact at all, FTIR measurements and BCA assays were conducted.

For FTIR measurements, samples from the PU prepolymer in ethyl acetate directly before dispersing and from the dispersions of the PUD with different amounts of hydrolyzed peptide directly after dispersing were vacuum-dried and measured immediately by analyzing the peak from the NCO band at around 2300 cm⁻¹. It was noted that there is no significant decrease in the NCO peak between the PU before emulsification and the PUD emulsified without peptide, indicating that the side-reaction of the NCO groups with water during emulsification is negligible. Also, a clear decrease in the NCO peak was seen for all dispersions containing peptide with regard to the dispersion formed without peptide. This general drop is due to the reaction occurring between the peptide mixture and the NCO groups. A noteworthy difference between the NCO peaks for emulsifications with the different amounts of peptide could not be detected. The results of the FTIR measurement are shown in **Figure 2**.

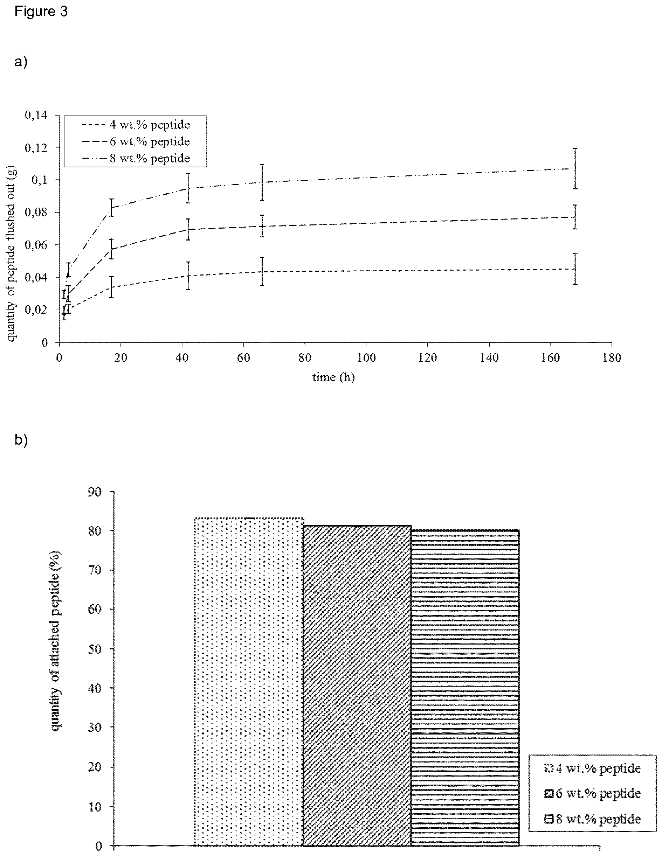

In order to complement this analysis, to quantify the adsorbance and reaction phenomena, the dispersions were dialyzed and the dialysis water batches were analysed using the BCA assay. This assay relies on the Biuret reaction between a peptide with alkaline Cu²⁺ to give a purple complex. It has been shown that the intensity of the complex increases proportionally with the amount of peptide present. Reference measurements of different quantities of hydrolysed peptide were also performed in order to allow for a quantification of the exact amounts of attached peptides. It was found that the relative fluorescence intensity measured at 590 nm increases linearly with the peptide concentration. **Figure 3** shows the results of the BCA assay. Values represent averages of three independent dialysis experiments and error bars represent the standard deviation.

While **Figure 3a** shows how much peptide was flushed out of the dispersion during dialysis at different points in time **Figure 3b** gives the overall total quantity of peptide attached to the particles for the different samples. **Figure 3b** displays the total amount of attached peptide. It can be seen that the amount of attached peptide increases with the amount of peptide present. This observation indicates that the interface was not yet saturated with peptide. Concerning the amount of peptide which can be covalently attached to the NCO groups, this observation is not surprising seen as there are still reactive NCO groups left after emulsification for all samples (see **Figure 2**). Concerning the amount of peptide adsorbed to the particles, it may also still be that the particle/water interface is not saturated with peptide yet: more peptide could possibly be adsorbed in order to permit for an even larger peptide corona.

### Particle Stability and Morphology

Another aspect was the investigation of the stabilization of the PUDs through the peptides. For the experiments, a PU backbone structure was selected which did not contain any moiety which could confer an electrostatic stabilization to the particles in order to avoid charge interactions with the peptides. As described above, most PUDs synthesized commonly contain negative charges due to sulfonate groups or pendant carboxylic acid groups which are very important for their stability. However, stabilization through the peptide component can also confer an additional property to the dispersions: by using stimuli-responsive peptides which precipitate through a slight change in pH, temperature, ionic strength, etc., one might be able to transfer this responsiveness to the particles.

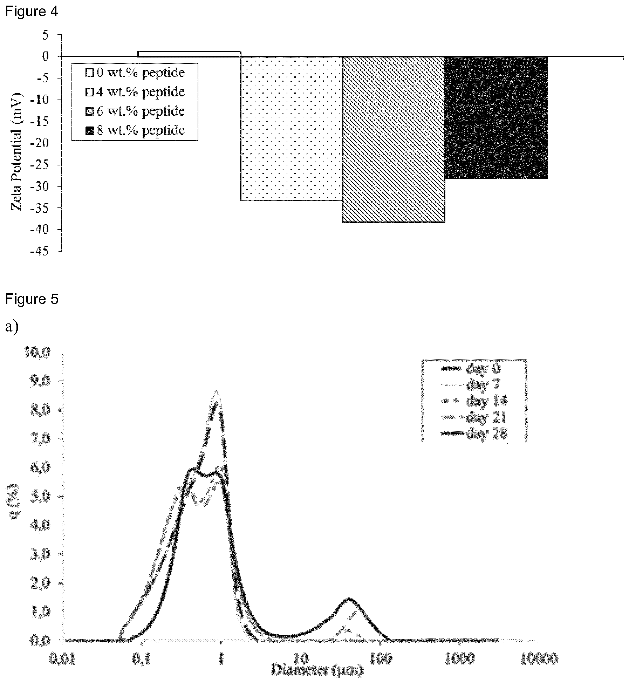

It has been shown that anionic stabilization can be bestowed by the peptides when using a mixture of peptides with low IPs. Thus, the Zeta Potential of the particles after dialysis was measured and the results are shown in **Figure 4**. It can clearly be seen that the peptides attached to the PUDs confer a negative charge to the particles whereas the particles without peptides are virtually uncharged. Furthermore, it can be noted that peptide-coated particles have Zeta Potentials ranging from -29 mV to -40 mV, which are sufficient to provide an initial, anionic stabilization for the particles. The differences in the exact values point towards the fact that the interaction between the peptides and the PUs is complex and highly dependent on the concentration of the dispersion solution, indicating that the system could be tuned further in order to adapt properties like colloidal stability, composition of the interface, etc. at will.

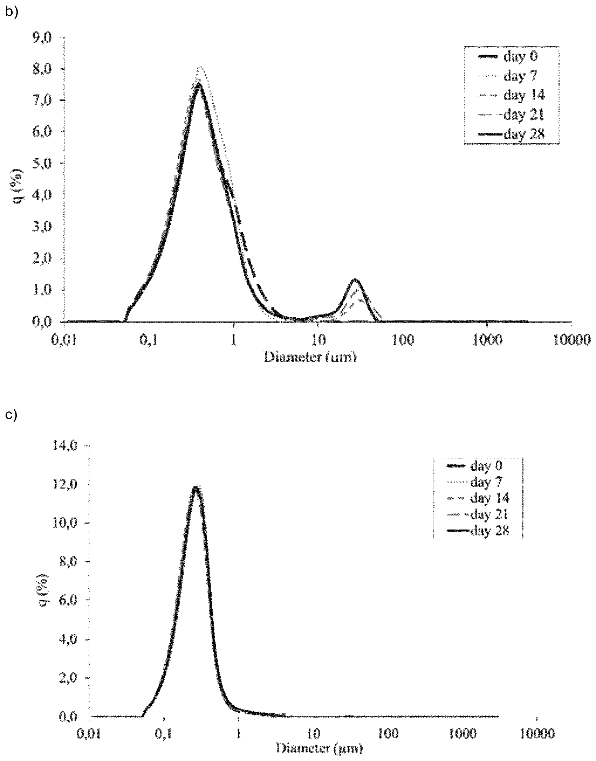

The particle stability was also investigated using laser diffraction measurements (LD). This technique was used rather than dynamic light scattering (DLS) in order to give a more precise measurement of the larger particles and agglomerates which may form overtime in the system. Measurements were carried out every week over the course of 1 month and results are displayed in **Figure 5**.

It was found that without any peptide, very large agglomerates with a diameter of about 1 mm resulted which were unstable and coagulated almost instantly. **Figure 5a** shows that with 4 wt.% hydrolyzed peptide, initially, particles of a median diameter of about 900 nm could be synthesized with a rather large size distribution which remained stable for one week without forming coagulates. After 14 days three different particle size peaks were measured, one of which corresponds to a very large size (> 10 µm) indicating that particles were destabilized and coalescence occurred. This trimodal size distribution is also seen in subsequent measurements. Furthermore, the peak at the largest diameter grows in proportion at the detriment of the two submicron peaks, showing that after 14 days, a stable dispersion is no longer present. When 6 wt. % hydrolyzed peptide were used in the synthesis (**Figure 5b**), the initial particle size is reduced to 400 nm, but the size distribution is rather large and at day 7 coagulation is observed during the measurement. With 8 wt.% peptide, however, the median particle size is reduced to 200 nm and the size distribution is narrow (**Figure 5c**). It is also highly interesting to notice that this small median particle size and narrow size distribution remain unchanged, even after one month. It can therefore be concluded that stable PUDs could be synthesized using 8 wt.% peptide with regard to the PU prepolymer.

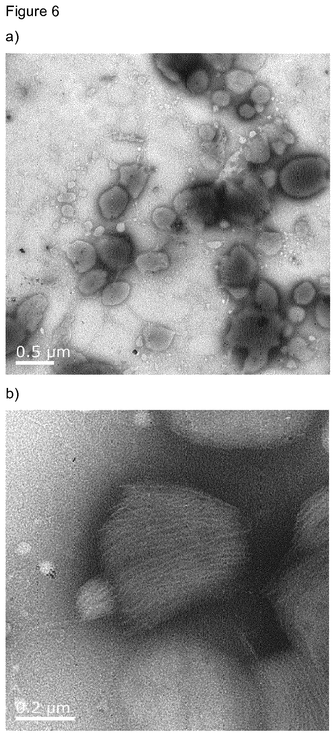

Furthermore, TEM was performed in order to investigate the particle morphology. As an example, results for the sample containing 8 wt.% peptide are displayed in **Figure 6**. **Figure 6a** shows the overall particle morphology and is consistent with the somewhat large size distribution observed in LD measurements. Also, the oval shape of the particles can be traced back to the low glass transition temperature of the PU's hard segments, which is commonly well below room temperature. Interestingly, within the particles, lamellar structures are visible, which are depicted in more detail in **Figure 6b**. These lamellar structures are most likely the first stages of crystallization of the polyesterrich segments

### Thermal Properties

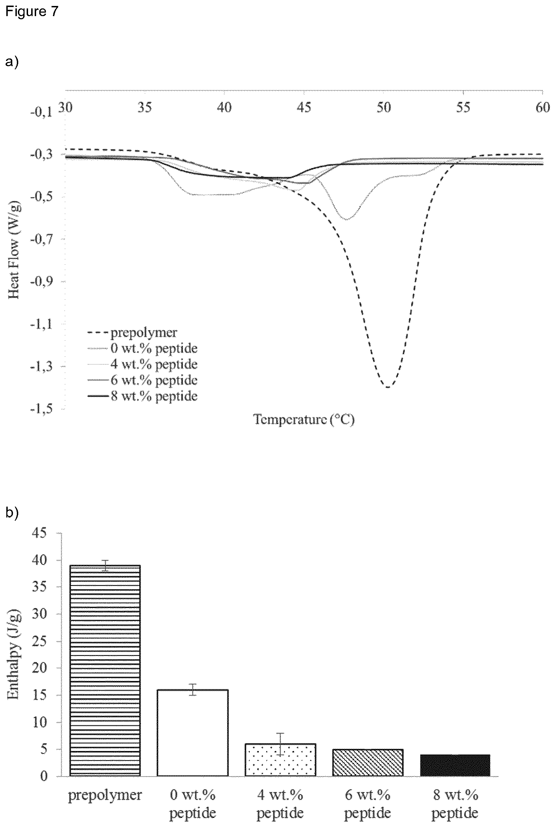

In order to further elucidate the thermal properties of the peptide-functionalized dispersions, DSC measurements of their respective films were carried out (**Figure 7**; Results are displayed as averages from two measurements. Error bars represent the standard deviation). Prior to this, the films were subjected to a heating and cooling protocol in order to create a uniform thermal history and thereby control their crystallization. **Figure 7a** shows the heat flow as a function of temperature. Strikingly, the prepolymer possesses the most pronounced and the narrowest melting peak (with a Tₘ of 50 °C), which is most likely due to the fact that the short PU prepolymer chains have the highest mobility in the film and therefore, the crystallites that are formed are very uniform in size. The sample that was dispersed without peptides, on the other hand, shows two melting peaks at lower temperatures (39 °C and 48 °C) which can be traced back to the fact that the dispersed sample hydrolysed completely. The longer PU chains are less mobile, an effect which is increased by the presence of urea linkages (formed during hydrolysis). Due to the stronger H-bonds formed by urea bonds with regard to urethane bonds, mobility in the film is further decreased and variations in crystal size and morphology can be expected. As a result, the melting peaks are numerous and broader. This general observation can also be made for the samples with peptide, however, in that case, the effect is less pronounced. Interestingly, the decrease in the melting enthalpy increases with the quantity of peptide present during dispersion, which is again indicative of a covalent attachment of the peptides taking place. It can be concluded that the peptides interfere even more with the crystallization that the urea bonds and this effect obviously increases with the quantity of attached peptide. This tendency is further underlined by the values of the melting enthalpy shown in **Figure 7b**. For the prepolymer, the measured enthalpy is 39 J/g, which is reduced to 16 J/g for the dispersed sample. The values for the peptide-containing samples are similar, decreasing from 6 to 6 J/g with increasing amount of peptide. Again, these results underline a decreased mobility in the film which is most likely due to an increased amount of covalently attached peptide.

## Claims

1. Process for manufacturing a peptide-stabilized polyurethane dispersion (PUD) comprising
(1) providing an NCO-terminated polyurethane prepolymer; and
(2) dispersing said NCO-terminated polyurethane prepolymer into a continuous aqueous phase, wherein the continuous aqueous phase is an aqueous solution comprising one or more peptides, thereby forming peptide-stabilized polyurethane particles.

2. The process of claim 1, wherein the aqueous solution comprises the one or more peptides in an amount of ≥ 4 wt.-%, preferably 4 to 20 wt.-%, more preferably 6 to 10 wt.-% relative to the total weight of the prepolymer.

3. The process of claim 1 or 2, wherein the aqueous solution has a pH greater than the isoelectric point of the one or more peptides, wherein the isoelectric point of the one or more peptides optionally is ≤7.

4. The process of any one of claims 1 to 3, wherein the one or more peptides are a peptide mixture, optionally obtainable by hydrolysis of a natural protein.

5. The process of any one of claims 1 to 4, wherein step (1) comprises forming an NCO-terminated polyurethane prepolymer from a reaction mixture comprising:
(a) at least one polyol, preferably with a number average molecular weight Mₙ in the range of 400 g/mol to 10000 g/mol; and
(b) at least one polyisocyanate, preferably at least one aliphatic di- and/or triisocyanate, wherein the at least one polyisocyanate is used in molar excess relative to the hydroxy groups of the at least one polyol of the reaction mixture to obtain an NCO-terminated polyurethane prepolymer.

6. The process of claim 5, wherein the at least one polyol comprises
(a) a polyether polyol; and/or
(b) a polyester polyol; and/or
(c) a polydimethylsiloxane polyol.

7. The process according to claim 5 or 6, wherein the reaction mixture comprises
(a) at least one crystalline or semicrystalline polyol, preferably polyester polyol, with a number average molecular weight Mₙ in the range of 400 g/mol to 5000 g/mol, a crystallinity of at least 20 % , preferably 35 % and more preferably 50% and a melting temperature Tₘ in the range of 30°C to 150°C, preferably 40 - 120 °C and more preferably 40 - 80 °C, the crystallinity and the melting temperature as determined by differential scanning calorimetry (DSC) according to ISO11357; and optionally
(b) at least one amorphous polyol, preferably polyether polyol, with a number average molecular weight Mₙ in the range of 400 g/mol to 5000 g/mol, a crystallinity of less than 10 % and a glass transition temperature T_{g} in the range of -60°C to 40°C, preferably -40°C to 30°C, the crystallinity and the glass transition temperature as determined by differential scanning calorimetry (DSC) according to ISO11357, wherein the molar ratio of the polyol (a) to the polyol (b) is in the range of 1:10 to 10:1.

8. The process according to any one of claims 5 to 7, wherein
(1) the at least one polyisocyanate is used in molar excess relative to the hydroxy groups of the combined polyols, the OH/NCO equivalent ratio preferably being 1:1.1 to 1:4, and/or
(2) the at least one polyisocyanate is at least one diisocyanate or triisocyanate, preferably selected from the group consisting of isophorone diisocyanate (IPDI), hexamethylene diisocyanate (HDI), polymeric polyisocyanates based on IPDI or HDI, and mixtures thereof.

9. The process of any one of claims 1 to 8, wherein the NCO-terminated polyurethane prepolymer
(a) is provided in form of a solution in an organic solvent; and/or
(b) has an average number molecular weight Mₙ of 3000 to 12000, preferably 4000 to 6000 g/mol; and/or
(c) comprises crystalline segments; and/or
(d) is essentially free of electrically charged groups or monomeric units.

10. The process according to any one of claims 1 to 9, wherein step (2) comprises
(a) emulsifying the polyurethane prepolymer into the continuous aqueous phase to form a pre-emulsion; and
(b) homogenizing the pre-emulsion to form a stable miniemulsion.

11. The process according to any one of claims 1 to 10, wherein the dispersing step (2), preferably the homogenization step,
(a) is carried out with a high shear process, preferably with a high shear process with (i) shear rates of at least 1,000,000/s, and/or (ii) an application of energy per time of at least 10³ J per second per liter of the emulsion; and/or
(b) comprises dispersing the prepolymer or prepolymer solution into the continuous aqueous phase under application of shear forces to obtain an emulsion with droplet sizes of between 50 and 500 nm, preferably 100 and 400 nm.

12. The process of any one of claims 1 to 11, wherein the process further comprises the step of (3) separating the peptide-stabilized polyurethane polymer particles from the unreacted peptides, preferably by dialysis.

13. The process of any one of claims 1 to 12, wherein
(a) the peptide-stabilized polyurethane polymer particles are polyurethane particles having a peptide corona; and/or
(b) essentially no additional stabilizer molecules different from the peptides are used or the amount of such additional stabilizer molecules used is lower than that needed to obtain polyurethane-based o/w-emulsions or dispersions with colloidal stability in the absence of the peptides, even when high shear forces are applied.

14. Aqueous peptide-stabilized polyurethane dispersion obtainable according to a process of any one of claims 1 to 13.

15. Composition, preferably cosmetic or adhesive composition, comprising the aqueous peptide-stabilized polyurethane dispersion according to claim 14.

16. Use of the aqueous peptide-stabilized polyurethane dispersion according to claim 14 in a cosmetic or adhesive composition.

## Patentansprüche

1. Verfahren zum Herstellen einer peptidstabilisierten Polyurethan-Dispersion (PUD), umfassend:
(1) Bereitstellen eines NCO-terminierten Polyurethan-Prepolymers; und
(2) Dispergieren des NCO-terminierten Polyurethan-Prepolymers in eine kontinuierliche wässrige Phase, wobei die kontinuierliche wässrige Phase eine wässrige Lösung ist, die ein oder mehrere Peptide umfasst, wodurch peptidstabilisierte Polyurethan-Partikel gebildet werden.

2. Verfahren nach Anspruch 1, wobei die wässrige Lösung das eine oder die mehreren Peptide in einer Menge von ≥ 4 Gew.-%, bevorzugt 4% bis 20 Gew.-%, besonders bevorzugt 6% bis 10 Gew.-%, bezogen auf das Gesamtgewicht des Prepolymers enthält.

3. Verfahren nach Anspruch 1 oder 2, wobei die wässrige Lösung einen pH-Wert aufweist, der größer ist als der isoelektrische Punkt des einen oder der mehreren Peptide, wobei der isoelektrische Punkt des einen oder der mehreren Peptide gegebenenfalls ≤7 ist.

4. Verfahren nach einem der Ansprüche 1 bis 3, wobei das eine oder die mehreren Peptide eine Peptidmischung sind, die gegebenenfalls durch Hydrolyse eines natürlichen Proteins erhältlich ist.

5. Verfahren nach einem der Ansprüche 1 bis 4, wobei Schritt (1) Bilden eines NCO-terminierten Polyurethan-Präpolymers aus einem Reaktionsgemisch umfasst, umfassend:
(a) mindestens ein Polyol, bevorzugt mit einem zahlengemittelten Molekulargewicht Mₙ im Bereich von 400 g/mol bis 10.000 g/mol; und
(b) mindestens ein Polyisocyanat, bevorzugt mindestens ein aliphatisches Di- und/oder Triisocyanat, wobei das mindestens eine Polyisocyanat bezogen auf die Hydroxy-Gruppen des mindestens einen Polyols der Reaktionsgemisch molarem Überschuss verwendet wird, um ein NCO-terminiertes Polyurethan-Prepolymer zu erhalten.

6. Verfahren nach Anspruch 5, wobei das mindestens eine Polyol umfasst:
(a) ein Polyetherpolyol; und/oder
(b) ein Polyesterpolyol; und/oder
(c) ein Polydimethylsiloxanpolyol.

7. Verfahren nach Anspruch 5 oder 6, wobei das Reaktionsgemisch umfasst:
(a) mindestens ein kristallines oder semikristallines Polyol, bevorzugt Polyesterpolyol, mit einem zahlengemittelten Molekulargewicht Mₙ im Bereich von 400 g/mol bis 5.000 g/mol, einer Kristallinität von mindestens 20%, bevorzugt 35% und mehr bevorzugt 50% und einer Schmelztemperatur Tₘ im Bereich von 30° bis 150 °C, bevorzugt 40°- 120 °C und mehr bevorzugt 40°- 80 °C, wobei die Kristallinität und die Schmelztemperatur mit Hilfe der Differentialscanningkalorimetrie (DSC) nach ISO 11357 bestimmt werden; und gegebenenfalls
(b) mindestens ein amorphes Polyol, bevorzugt Polyetherpolyol, mit einem zahlengemittelten Molekulargewicht Mₙ im Bereich von 400 g/mol bis 5.000 g/mol, einer Kristallinität von weniger als 10% und einer Glasübergangstemperatur T_{g} im Bereich von -60 °C bis 40 °C, bevorzugt -40 °C bis 30 °C, wobei die Kristallinität und die Glasübergangstemperatur mit Hilfe der Differentialscanningkalorimetrie (DSC) nach ISO 11357 bestimmt werden, wobei das Molverhältnis des Polyols (a) zu dem Polyol (b) im Bereich von 1:10 bis 10:1 liegt.

8. Verfahren nach einem der Ansprüche 5 bis 7, wobei
(1) das mindestens eine Polyisocyanat bezogen auf die Hydroxy-Gruppen der vereinigten Polyole im molarem Überschuss eingesetzt wird, wobei das OH/NCO-Äquivalentverhältnis bevorzugt 1:1,1 bis 1:4 beträgt, und/oder
(2) das mindestens eine Polyisocyanat mindestens ein Diisocyanat oder Triisocyanat ist, bevorzugt ausgewählt aus der Gruppe bestehend aus Isophorondiisocyanat (IPDI), Hexamethylendiisocyanat (HDI), polymeren Polyisocyanaten auf Basis von IPDI oder HDI und Mischungen davon.

9. Verfahren nach einem der Ansprüche 1 bis 8, wobei das NCO-terminierte Polyurethan-Prepolymer
(a) bereitgestellt wird in Form einer Lösung in einem organischen Lösungsmittel; und/oder
(b) ein zahlengemitteltes Molekulargewicht Mₙ von 3.000 bis 12.000, bevorzugt von 4.000 bis 6.000 g/mol aufweist; und/oder
(c) kristalline Segmente umfasst; und/oder
(d) weitgehend frei von elektrisch geladenen Gruppen oder Monomereinheiten ist.

10. Verfahren nach einem der Ansprüche 1 bis 9, wobei Schritt (2) umfasst:
(a) Emulgieren des Polyurethan-Präpolymers in die kontinuierliche wässrige Phase, um eine Voremulsion zu bilden; und
(b) Homogenisieren der Voremulsion, um eine stabile Mini-Emulsion zu bilden.

11. Verfahren nach einem der Ansprüche 1 bis 10, wobei der Schritt des Dispergierens (2), bevorzugt der Homogenisierungsschritt,
(a) mit einem Prozess mit hoher Scherung ausgeführt wird, bevorzugt mit einem Prozess mit hoher Scherung mit
(i) Schergeschwindigkeiten von mindestens 1.000.000/s und/oder
(ii) eine Energiezufuhr pro Zeit von mindestens 10³ J pro Sekunde pro Liter der Emulsion; und/oder
(b) Dispergieren der Prepolymer oder Prepolymer-Lösung in die kontinuierliche wässrige Phase unter Anwendung von Scherkräften umfasst, um eine Emulsion mit Tröpfchengrößen zwischen 50 und 500 nm, bevorzugt 100 und 400 nm, zu erhalten.

12. Verfahren nach einem der Ansprüche 1 bis 11, wobei das Verfahren ferner den Schritt (3) des Trennens der peptidstabilisierten Polyurethanpolymerteilchen von den nicht umgesetzten Peptiden, bevorzugt durch Dialyse, umfasst.

13. Verfahren nach einem der Ansprüche 1 bis 12, wobei
(a) die peptidstabilisierten Polyurethanpolymerteilchen Polyurethanteilchen mit einer Peptidkorona sind; und/oder
(b) weitgehend keine zusätzlichen Stabilisatormoleküle verwendet werden, die von den Peptiden verschieden sind, oder die Menge solcher zusätzlichen verwendeten Stabilisatormoleküle geringer ist als diejenige, die notwendig ist, um auf PolyurethanBasis verwendete O/W-Emulsionen oder Dispersionen mit kolloidaler Stabilität in Abwesenheit der Peptide zu erhalten, selbst wenn hohe Scherkräfte angewendet werden.

14. Wässrige peptidstabilisierte Polyurethandispersion, die mit einem Verfahren nach einem der Ansprüche 1 bis 13 erhalten werden kann.

15. Zusammensetzung, bevorzugt kosmetische oder adhäsive Zusammensetzung, umfassend die wässrige peptidstabilisierte Polyurethandispersion nach Anspruch 14.

16. Verwendung der wässrigen peptidstabilisierten Polyurethandispersion nach Anspruch 14 in einer kosmetischen oder adhäsiven Zusammensetzung.

## Revendications

1. Processus de fabrication d'une dispersion de polyuréthane stabilisé par voie peptidique (PUD) comprenant
(1) l'utilisation d'un prépolymère de polyuréthane à terminaison NCO ; et
(2) la dispersion dudit prépolymère de polyuréthane à terminaison NCO dans une phase aqueuse continue, la phase aqueuse continue étant une solution aqueuse comprenant un ou plusieurs peptides, formant ainsi des particules de polyuréthane stabilisé par voie peptidique.

2. Processus selon la revendication 1, dans lequel la solution aqueuse comprend l'au moins un peptide en une quantité supérieure ou égale à 4 % en poids, de préférence de 4 à 20 % en poids, plus préférablement de 6 à 10 % en poids par rapport au poids total du prépolymère.

3. Processus selon la revendication 1 ou 2, dans lequel la solution aqueuse a un pH supérieur au point isoélectrique de l'au moins un peptide, le point isoélectrique de l'au moins un polypeptide étant éventuellement inférieur ou égal à 7.

4. Processus selon l'une quelconque des revendications 1 à 3, dans lequel l'au moins un polypeptide est un mélange de peptides, pouvant éventuellement être obtenu par hydrolyse d'une protéine naturelle.

5. Processus selon l'une quelconque des revendications 1 à 4, dans lequel l'étape (1) comprend la formation d'un prépolymère de polyuréthane à terminaison NCO à partir d'un mélange réactionnel comprenant :
(a) au moins un polyol, de préférence ayant une masse moléculaire moyenne en nombre Mₙ dans la plage allant de 400 g/mol à 10 000 g/mol ; et
(b) au moins un polyisocyanate, de préférence au moins un di- et/ou triisocyanate aliphatique, l'au moins un polyisocyanate étant utilisé en excès molaire par rapport aux groupes hydroxy de l'au moins un polyol du mélange réactionnel afin d'obtenir un prépolymère de polyuréthane à terminaison NCO.

6. Processus selon la revendication 5, dans lequel l'au moins un polyol comprend
(a) un polyol de polyéther ; et/ou
(b) un polyol de polyester ; et/ou
(c) un polyol de polydiméthylsiloxane.

7. Processus selon la revendication 5 ou 6, dans lequel le mélange réactionnel comprend
(a) au moins un polyol cristallin ou semi-cristallin, de préférence un polyol de polyester, de masse moléculaire moyenne en nombre Mₙ dans la plage allant de 400 g/mol à 5 000 g/mol, une cristallinité d'au moins 20 %, de préférence de 35 % et plus préférablement de 50 %, et une température de fusion Tₘ dans la plage comprise entre 30 °C et 150 °C, de préférence entre 40 et 120 °C et plus préférablement entre 40 et 80 °C, la cristallinité et la température de fusion telles que déterminées par calorimétrie différentielle à balayage (DSC) conformément à ISO 11357 ; et éventuellement
(b) au moins un polyol amorphe, de préférence un polyol de polyéther, de masse moléculaire moyenne en nombre Mₙ dans la plage allant de 400 g/mol à 5 000 g/mol, une cristallinité inférieure à 10 % et une température de transition vitreuse T_{g} dans la plage comprise entre -60 °C et 40 °C, préférablement entre -40 °C et 30 °C, la cristallinité et la température de transition vitreuse telles que déterminées par calorimétrie différentielle à balayage (DSC) conformément à la norme ISO 11357, le rapport molaire du polyol (a) au polyol (b) étant dans la plage allant de 1:10 à 10:1.

8. Processus selon l'une quelconque des revendications 5 à 7, dans lequel
(1) l'au moins un polyisocyanate est utilisé en excès molaire par rapport aux groupes hydroxy combinés, le rapport équivalent OH/NCO étant de préférence 1:1,1 à 1:4, et/ou
(2) l'au moins un polyisocyanate est au moins un diisocyanate ou un triisocyanate, de préférence choisi dans l'ensemble constitué du diisocyanate d'isophorone (IPDI), du diisocyanate d'hexaméthylène (HDI), de polyisocyanates polymériques à base d'IPDI ou de HDI, et de leurs mélanges.

9. Processus selon l'une quelconque des revendications 1 à 8, dans lequel le prépolymère de polyuréthane à terminaison NCO
(a) est utilisé sous la forme d'une solution dans un solvant organique ; et/ou
(b) a une masse moléculaire moyenne en nombre Mₙ dans la plage comprise entre 3 000 et 12 000, de préférence entre 4 000 et 6 000 g/mol ; et/ou
(c) comprend des segments cristallins ; et/ou
(d) est essentiellement exempt de groupes électriquement chargés ou d'unités monomériques.

10. Processus selon l'une quelconque des revendications 1 à 9, dans lequel l'étape (2) comprend
(a) l'émulsion du prépolymère de polyuréthane dans la phase aqueuse continue pour former une pré-émulsion ; et
(b) l'homogénéisation de la pré-émulsion pour former une mini-émulsion stable.

11. Processus selon l'une quelconque des revendications 1 à 10, dans lequel l'étape de dispersion (2), de préférence l'étape d'homogénéisation,
(a) est effectuée selon un processus de cisaillement élevé, de préférence selon un processus de cisaillement élevé avec (i) des taux de cisaillement d'au moins 1 000 000/s, et/ou (ii) une application d'énergie en fonction du temps d'au moins 10³ J par seconde par litre d'émulsion ; et/ou
(b) comprend la dispersion du prépolymère ou de la solution de prépolymère dans la phase aqueuse continue sous l'application de forces de cisaillement afin d'obtenir une émulsion dont les tailles de gouttelettes sont comprises entre 50 et 500 nm, de préférence comprise entre 100 et 400 nm.

12. Processus selon l'une quelconque des revendications 1 à 11, dans lequel le processus comprend en outre l'étape de (3) séparation entre les particules polymère de polyuréthane stabilisé par voie peptidique et les peptides n'ayant pas réagi, de préférence par dialyse.

13. Processus selon l'une quelconque des revendications 1 à 12, dans lequel
(a) les particules polymère de polyuréthane stabilisé par voie peptidique sont des particules de polyuréthane comportant une couronne de peptide ; et/ou
(b) essentiellement aucune molécule stabilisatrice supplémentaire différente des peptides n'est utilisée, ou la quantité de ces molécules stabilisatrices supplémentaires utilisées est inférieure à celle nécessaire pour obtenir des émulsions ou des dispersions à base de polyuréthane présentant une stabilité colloïdale en l'absence des peptides, même lorsque des forces élevées de cisaillement sont appliquées.

14. Dispersion de polyuréthane aqueux stabilisé par voie peptidique pouvant être obtenue conformément à un processus selon l'une quelconque des revendications 1 à 13.

15. Composition, de préférence composition cosmétique ou adhésive, comprenant la dispersion de polyuréthane aqueux stabilisé par voie peptidique selon la revendication 14.

16. Utilisation de la dispersion de polyuréthane aqueux stabilisé par voie peptidique selon la revendication 14 dans une composition cosmétique ou adhésive.
